# EUROPÄISCHE PATENTSCHRIFT

(11) **EP 0 655 002 B1**
(45) Veröffentlichungstag und Bekanntmachung des Hinweises auf die Patenterteilung: **28.01.1998**
(21) Anmeldenummer: 93917549.3
(22) Anmeldetag: 12.08.1993
(51) Int. Cl.: A61L 29/00, A61L 27/00, A61L 31/00

(54) **BAKTERIZIDE UND/ODER FUNGIZIDE KUNSTSTOFFGEGENSTÄNDE FÜR DEN MEDIZINISCHEN BEDARF**
BACTERICIDAL AND/OR FUNGICIDAL PLASTIC OBJECTS FOR MEDICAL USES
OBJETS BACTERICIDES ET/OU FONGICIDES EN MATIERES PLASTIQUES A USAGE MEDICAL

(30) Priorität: 13.08.1992 DE 4226810
(43) Veröffentlichungstag der Anmeldung: 31.05.1995
(73) Patentinhaber: KRALL, Theodor, A-6600 Lechaschau (AT); GUGGENBICHLER, J. Peter, D-90742 Fürth (DE)
(72) Erfinder: KRALL, Theodor, A-6600 Lechaschau (AT); GUGGENBICHLER, J., Peter, D-90742 Fürth (DE); GIRISCH, Monika, D-92637 Weiden (DE)
(74) Vertreter: VOSSIUS & PARTNER
(86) Internationale Anmeldenummer: DE9300726
(87) Internationale Veröffentlichungsnummer: WO9404202

(56) Entgegenhaltungen:
- EP-A- 0 550 875
- WO-A-87/03495
- WO-A-89/04682
- DE-A- 1 939 687
- FR-A- 2 209 582
- DATABASE WPI Week 9241, Derwent Publications Ltd., London, GB; AN 92-337629 & JP,A,4 243 908 (RASA KOGYO KK) 1. September 1992

## Beschreibung

Die Erfindung betrifft Kunststoffgegenstände, insbesondere für den medizinischen Bedarf, die so langzeitig keine Besiedelung mit Keimen und/oder Pilzen ermöglichen, daß sie für den Patienten eine klare Risikominderung der Infektion mit Keimen pathogener Art für die ganze Dauer der Anwendung erbringen.

Nach dem heutigen Stand des Angebotes kommen eine Vielzahl von Produkten für den medizinischen Bedarf in den Handel, die einen Kunststoffbestandteil aufweisen. Bei einer Reihe von Produkten ist ein wesentlicher Teil ein Schlauch mit einem oder mehreren Lumen. Diese Produkte werden sterilisiert, z.B. mit Ethylenoxid, und gelangen luftdicht verpackt in den Handel.

Entnimmt man sie der Verpackung, so bleibt die Sterilität jedoch nicht erhalten. Zwar werden für derartige Produkte bevorzugt dichte und glatte Materialien verwendet, die die Besiedelung mit Bakterien und Pilzen hintan halten sollen. Insbesondere bei längerem oder gar langzeitigem Gebrauch können diese Produkte aber doch wieder von Keimen besiedelt werden, die letzlich dem Patienten, an dem diese Produkte verwendet werden, schaden.

Versuche, Produkte der eingangs beschriebenen Art mit Antibiotika, auch in Verbindung mit metallhaltigen Verbindungen, zu versetzen oder Antibiotika enthaltende Beschichtungen gebrauchstauglich zu machen, haben offenbar zumindest bis jetzt nicht zum gewünschten Ergebnis geführt, z.B. US-A-4 612 337. Vor allem besteht dabei durch die Langzeit-Abgabe von Antibiotika in geringer Konzentration die Gefahr der Resistenzbildung.

Von weiterer Bedeutung ist die antimikrobielle Eigenschaft von Silber. Bereits Spuren von Silber und seinen Salzen zeigen eine bakteriostatische und bakterizide Wirkung. Diese desinfizierende Wirkung ist auf das von C. von Naegli (1893) bezeichnete Phänomen der "Oligodynamie" zurückzuführen, welches auf der in Lösung gehenden Silberionen beruhen soll. Nach diesem Prinzip arbeitet auch das "Mittel zum Entseuchen keimhaltiger Flüssigkeiten" in der DE-PS 900 000. Das Mittel besteht aus einem Komplexsalz, zusammengesetzt aus einem Schwermetallsalz mit spezifischer oligodynamischer Wirkung (z.B. Ag) und einem zur Komplexsalzbildung befähigten löslichen Halogenid, einem leicht löslichen Salz eines Metalls oder einer organischen Base.

DE-A-37 25 728, EP-A-0 301 717 und US-A-4 054 139 beschreiben medizinische Polymermaterialien, bei denen Silber(verbindungen) mechanisch in den Werkstoff eingearbeitet werden. Auch bei diesen Materialien kann keine ausreichend lange Sterilität erreicht werden. Ebenso hat der Versuch, derartige Produkte mit stark bakterizidem Silbernitrat zu behandeln, um längerzeitige Sterilität herbeizuführen, zu keinem positiven Ergebnis geführt. Vermutlich wurde das Silbernitrat aufgrund seiner Wasserlöslichkeit einfach ausgewaschen.

Ebenso ist die bakterizide und/oder fungizide Wirkung von anderen Metallen hinreichend bekannt. So z.B. von Kupfer, das in der EP-A-0 116 865 beschrieben wird und das insbesondere auch eine starke fungizide Wirkung zeigt sowie von Gold, Zink und Cer, was auch in der US-A-4 612 332 offenbart wird.

Es gelang jedoch auch mit diesen Metallen bislang nicht, zufriedenstellende Ergebnisse bei der Imprägnierung von Kunststoffteilen zu erzielen.

Ebenfalls ist die bakterizide und/oder fungizide Wirkung dieser Metalle nicht ausreichend, um allen Anforderungen der Praxis zu genügen. Vielmehr besteht ein Bedarf nach Mitteln auf der Grundlage solcher Metalle, in denen jedoch die bakterizide und/oder fungizide Wirksamkeit dieser Metalle gesteigert ist.

Es ist Aufgabe der Erfindung, ein Verfahren zur Verfügung zu stellen, mit dem es möglich ist, Kunststoffteile vorzugsweise zur Verwendung in der Medizin herzustellen, die eine sehr hohe bakterizide und/oder fungizide Wirksamkeit auch nach längerer, nicht steriler Anwendung an Mensch oder Tier aufweisen.

Diese Aufgabe wird gelöst durch ein Verfahren zur Herstellung eines nicht von Keimen besiedelbaren Kunststoffteils, das dadurch gekennzeichnet ist, daß der Kunststoff mit einem Quellungsmittel vorbehandelt wird und der erhaltene gequollene Kunststoff mit einer Lösung aus einer in Wasser unlöslichen Verbindung, die ein bakterizides und/oder fungizides Metall enthält, behandelt wird.

Gegenstand der Erfindung sind ebenfalls die nach diesem Verfahren herstellbaren Kunststoffteile.

Bei den Verbindungen, die ein bakterizides und/oder fungizides Metall enthalten, handelt es sich um Verbindungen, die in Wasser unlöslich sind, da ansonsten eine erhöhte Gefahr besteht, daß das bakterizide und/oder fungizide Metall während der Anwendung am Menschen oder am Tier durch Körperflüssigkeit aus dem Kunststoffteil ausgespült wird und damit die langfristige Bakterizid- und/oder Fungizidwirksamkeit des Kunststoffteils herabgesetzt werden könnte.

EP-A-0 550 875 beschreibt ein ähnliches Verfahren und ein durch das Verfahren erhältliches Produkt, wobei aber auf die Verwendung von in Wasser unlöslichen Verbindungen nicht weiter eingegangen wird. Der Inhalt dieses Dokuments ist Stand der Technik gemäß Art. 54(3)/(4) EPÜ und wurde bei der Erstellung der Patentansprüche der vorliegenden Anmeldung berücksichtigt.

Ebenso ist es bevorzugt, daß es sich bei der metallhaltigen Verbindung um ein anorganisches Metallsalz handelt. Das Metall ist bevorzugt Silber, Kupfer, Gold, Zink oder Cer. Von diesen Metallen sind Silber und Kupfer und insbesondere Silber besonders bevorzugt. Vor allem mit Silberhalogeniden, wie Silberchlorid, und Kupfer(I)-halogeniden, wie Kupfer(I)-jodid lassen sich hervorragende Ergebnisse bei der Herstellung von nicht von Keimen besiedelbaren Kunststoffteilen erzielen. Es ist ebenfalls möglich, Mischungen aus Verbindungen mit verschiedenen Metallen einzusetzen. In dieser Hinsicht ist eine Mischung aus Kupfer(I)-jodid und Silberchlorid bevorzugt.

Als polymere Verbindungen für das nicht von Keimen besiedelbare Kunststoffteil kommen die in der Klinik üblicherweise verwendeten Kunststoffe in Betracht. Dies sind insbesondere Polyethylen, Polypropylen, vernetzte Polysiloxane, Polyurethane, Polymere auf (Meth)acrylatbasis, Cellulose und Cellulosederivate, Polycarbonate, ABS, Tetrafluorethylenpolymere und Polyethylenterephthalate, sowie die entsprechenden Copolymeren. Besonders bevorzugt sind Polyethylen und Polypropylen sowie Polyethylen-Polypropylen-Copolymere.

Die Kunststoffteile werden bevorzugt in medizinischen Geräten eingesetzt, die auf irgendwelche Art und Weise in den menschlichen oder tierischen Körper implantiert oder an ihm angewendet werden. Als solche Produkte kommen beispielsweise Shunts, Kannülen, Wundverbände, endotracheale Röhren, percutane Vorrichtungen, intraokulare Linsen, Kontaktlinsen, Nähte und beliebige sonstige, aus Kunststoffen herstellbare Implantate, insbesondere jedoch Katheter jedweder Art in Frage.

In einer besonders bevorzugten Ausführungsform wird der mit einem Quellungsmittel gequollepe Kunststoff ohne vorheriges Trocknen mit einer Lösung einer in Wasser unlöslichen Verbindung, die ein bakterizides und/oder fungizides Metall enthält, behandelt, wobei die ein bakterizides und/oder fungizides Metall enthaltende Verbindung in dem Lösungsmittel, in dem sie gelöst wurde, deutlich besser löslich ist als in dem Quellungsmittel, das zur Quellung des Kunststoffes verwendet wurde. Außerdem sollte das Lösungsmittel mit dem Quellungsmittel mischbar sein. Der Vorteil dieser Ausführungsform liegt darin, daß sich in dem gequollenen Kunststoff ein Konzentrationsgradient zwischen dem besseren Lösungsmittel für die ein bakterizides und/oder fungizides Metall enthaltende Verbindung und dem schlechteren Lösungsmittel für diese Verbindung einstellt. Die metallhaltige Verbindung diffundiert zunächst in dem besseren Lösungsmittel in das gequollene Kunststoffteil hinein, solange bis der Anteil an schlechterem Lösungsmittel überwiegt und die Verbindung in dem Kunststoffteil ausfällt. Da das hierdurch entstandene Konzentrationsgefälle in dem Lösungsgemisch wieder durch Diffusion ausgeglichen wird, ist es möglich, eine höhere Konzentration an bakterizidem und/oder fungizidem Metall in dem Kunststoffteil zu erzielen. Da sich ferner der Verteilungsgradient zwischen besserem Lösungsmittel für die Verbindung und schlechterem Lösungsmittel für die Verbindung kontinuierlich ändert, wird eine spezielle Verteilung (Gradient) des bakteriziden und/oder fungiziden Metalls in dem Kunststoffteil erreicht, die sich in der Praxis als ausgesprochen wirksam erwiesen hat, die Besiedelung dieses Kunststoffteils mit Keimen auch bei längerer Anwendung am Menschen oder Tier, und in Kontakt zu Körperflüssigkeiten zu verhindern. Wechselwirkungen zwischen dem Kunststoff und der Metallverbindung können beim Aufbau der Verteilung der Silberverbindung im Kunststoff ebenfalls eine Rolle spielen.

In einer abgewandelten Ausführungsform wird ein geeigneter Kunststoffgegenstand (z.B. ein Schlauch) von einer Seite mit dem Quellungsmittel und von der anderen Seite mit der ein bakterizides/fungizides Metall enthaltenden Lösung behandelt.

Es ist ebenfalls bevorzugt, daß das Lösungsmittel für die Verbindung, die ein bakterizides und/oder fungizides Metall enthält, in einem Lösungsmittel erfolgt, mit diesem Metall einen stabilen Komplex bilden kann. Zum einen hat sich nämlich gezeigt, daß die Verwendung von komplexierten Metallen ebenfalls zu einem äußerst günstigen Verteilungsprofil der ein bakterizides und/oder fungizides Metall enthaltenden Verbindung in dem Kunststoffteil führt, zum anderen wurde beobachtet, daß die bakterizide und/oder fungizide Wirksamkeit einzelner Metalle durch ihre Komplexierung in einem Lösungsmittel gesteigert werden kann.

Es wurde auch für eine Lösung einer metallhaltigen (insbesondere einer silberhaltigen) Verbindung in einem Alkanolamin auch gefunden, daß der entstehende Metall(Silber-)alkanolaminkomplex deutlich bessere bakterizide und/oder fungizide Wirksamkeit aufweist, als eine Lösung einer metall(silber-)haltigen Verbindung in einem nichtkomplexierenden Lösungsmittel.

Für diese ausgesprochen stark bakterizid wirksame Mittel sind im medizinischen Bereich jedoch auch weitere Anwendungen denkbar, bei denen es auf eine besonders hohe bakterizide und/oder fungizide Wirksamkeit ankommt. Insbesondere sind die erfindungsgemäßen Mittel günstig zur Bekämpfung von grampositiven oder gramnegativen Bakterien wie S. aureus, S. faecalis, Grp. B. strep, E. coli, Klebsiella, Proteus, Salmonella, Enterobacter, Serratia, P. aeruginosa, Acinetobacter.

Unter den vorstehend beschriebenen Gesichtspunkten sind besonders bevorzugte Quellungsmittel niedere Alkohole mit nicht mehr als 10 Kohlenstoffatomen, wie Methanol, Ethanol, Propanol, Isopropanol und die vier isomeren Butanole, insbesondere Ethanol, sowie üblicherweise als Lösungsmittel verwendete niedere Ketone, wie Methylethylketon oder Aceton, insbesondere Aceton. Chlorierte Kohlenwasserstoffe sind zwar nicht bevorzugt, können nach Maßgabe des Fachmanns jedoch auch verwendet werden.

Für die Lösungsmittel sind organische Amine bevorzugt, insbesondere Alkanolamine mit bis zu 10 Kohlenstoffatomen und vor allem Ethanolamin. Auch Diethanolamin und Triethanolamin werden bevorzugt verwendet.

Vorzugsweise werden bei Raumtemperatur gesättigte Lösungen des Metallsalzes im Lösungsmittel hergestellt.

Damit ergibt sich als eine der bevorzugten Ausführungsformen eine Lösung eines anorganischen Silber- oder Kupfer(I)-salzes, bevorzugt Silberchlorid und/oder Kupfer(I)-jodid in einem Alkanolamin, bevorzugt Ethanolamin, das zusammen mit einem Quellungsmittel verwendet wird, wobei als Quellungsmittel bevorzugt Ethanol und/oder Aceton verwendet wird.

Das Verfahren wird besonders vorteilhaft wie folgt durchgeführt:

Zunächst wird das Silber- oder Kupfer(I)-salz in hoher Konzentration (d.h. nahe der Sättigungsgrenze) in Ethanolamin gelöst und der Kunststoff mit einem Quellungsmittel behandelt, das mit dem vorgenannten Lösungsmittel für das Silber- oder Kupfer(I)-salz mischbar ist, aber seinerseits das Silber- oder Kupfer(I)-salz kaum löst (z.B. Aceton oder auch Ethanol). Wird jetzt der gequollene Kunststoff mit der Silbersalz- oder der Kupfer(I)-salzlösung in Kontakt gebracht, so diffundiert damit auch Silber- oder Kupfer(I)-salz in den Kunststoff ein und wird dabei aus dem Gemisch der Lösungsmittel ausgefällt. Durch nachfolgende Trocknung kann das Silber- oder Kupfersalz im Kunststoff haltbar gespeichert werden. Dieser Vorgang kann schon im Vorprodukt für das Fertigprodukt, im Kunststoffgranulat, durchgeführt werden.

Das Beispiel erläutert die Erfindung. Falls im folgenden nicht anders erwähnt, folgte die Imprägnierung mit einer Lösung aus Silberchlorid in Ethanolamin beim Raumtemperatur.

### Beispiel

### Behandlung eines Schlauchs (Katheters) mit AgCl/H₂NCH₂CH₂OH

Eine besonders wirkungsvolle Vorgangsweise ist in der Form erprobt worden, daß ein HDPE-Schlauch in Aceton einen Tag gelagert und damit wenigstens im Rahmen des Möglichen zum Quellen gebracht worden ist. PU-Schläuche sind in etwa 2 Stunden ausreichend gequollen und werden dann weiterverarbeitet. Er wurde danach schnell entleert und mit einer Lösung von Silberchlorid in Ethanolamin gefüllt. Das in der Wandung des Schlauches befindliche Aceton konnte nach außen wegdunsten. Der auf diese Weise behandelte Schlauch wurde danach insgesamt noch einmal mit der Silbersalzlösung behandelt, um auch außen eine hinreichend wirksame Beschichtung mit Silberchlorid zu erzielen. Insgesamt betrug die Aufnahme von Silberchlorid weniger als 0,5 % des Gewichtes des Schlauches.

### Herstellung der Keimsuspension für den Test auf Keimbesiedelung

Als Keimsuspension wurde 5 %ige Glucoselösung mit Staphylokokkus epidermidis in einer Größenordnung von zwei kalibrierten Ösen Staphylokokkus epidermidis von der Blutagarplatte pro einem Liter Glucose beimpft. Es handelt sich dabei um etwa 500 000 Keime/ml.
Da Glucose eine leicht bakterizide Wirkung hat, wurde die Keimsuspension täglich nachgeimpft, um eine gleichbleibend große Anzahl von Keimen zu gewährleisten. Die Keimbesiedlung und die Inokulumdichte wurden regelmäßig überprüft.
Zur Aufbewahrung wurde der Keim in ein steriles Kryogefäß überimpft und bei -60°C gelagert. Ein solches Kryogefäß enthält mehrere Kügelchen, an denen Mikroorganismen vollständig binden. Wurde der Keim benötigt, setzte man eine neue Kultur an, indem ein Kügelchen auf einer Blutagarplatte ausgerollt und die Kultur anschließend 24 Stunden lang bei 37°C inkubiert wurde.

### Test auf Keimbesiedelung

Die Katheter wurden zuerst mit Ethylenoxyd sterilisiert und anschließend mit der oben beschriebenen Keimsuspension bei einer Fließgeschwindigkeit von 10 ml/Stunde durchspült. Die Durchflußrate wurde bei den ersten Versuchen mit einer Peristaltikpumpe, später zur gleichzeitigen Testung mehrerer Katheter, mit Helix-Reglersystemen begrenzt.

Im Abstand von sechs bis acht Stunden, später alle zwölf Stunden, wurden die Katheter mit 100 ml 0,9 %iger Kochsalzlösung über einen Dreiwegehahn gespült und anschließend ein ca. 1 cm langes Katheterstück mit einer sterilen Schere abgeschnitten. Dieses Katheterstück wurde in 2 ml Todd Hewitt Broth eingelegt und mindestens 48 Stunden bei 37°C inkubiert.
Bei Trübwerden der Nährbouillon galt der Katheter als kontaminiert. Die Katheter wurden bis zur Kontamination bzw. bei erhaltener Sterilität 200 Stunden lang mit der Keimsuspension durchspült.
Die antimikrobielle Wirkung der silberimprägnierten Katheter wurde mit der Keimbesiedlung unbehandelter Kontrollkatheter in einem Doppelblind-Verfahren verglichen.

### Gewebeverträglichkeit

Bei dieser Untersuchung wurde der Einfluß der verschiedenen Kathetermaterialien auf die Vitalität Phytohämagglutininstimulierter Lymphozyten bestimmt.
Nach Messung der Lymphozytenkulturen im Photometer bei einer optischen Dichte von 550 nm, konnte anschließend der Anteil noch lebender Lymphozyten in Prozent bestimmt werden.

### Thrombogenitätsprüfung

Pro Thrombogenitätsprüfung wurde ein silberimprägnierter Katheter aus Pellethan acht Stunden lang mit Frischblut, versehen mit 2,5 ml ACD-Stabilisator Pro 10 ml Vollblut, durchströmt. Die Fließgeschwindigkeit durch den Katheter betrug 5,5 ml pro Stunde. Jeweils 3,5 ml vom Ausgangsblut und 3,5 ml vom Blut, welches den Katheter passiert hat, wurden im Abstand von zwei Stunden entnommen und auf die Gerinnungswerte Quick, PTT, TZ, Fibrinogengesamtkonzentration und Thrombinzeit untersucht.

### Ergebnisse der antimikrobiellen Wirksamkeit

### Polyethylen/Polypropylen (PE/PP):

- unbehandelter Kontrollkatheter: nach 18 Stunden kontaminiert
- silberimprägnierter Katheter (Quellung mit Aceton): 168 Stunden lang steril*

### Polyurethan (PU):

a) PU I (Cavafix®, hergestellt aus Pellethane®):
- unbehandelter Kontrollkatheter: nach 24 Stunden kontaminiert
- silberimprägnierter Katheter:

| Quellmittel | Imprägn. innen in Minuten | Imprägn. außen in Min. | Ergebnis |
|---|---|---|---|
| Isopropanol | 195 | 120 | nach 350 Std. steril* |
| Ethanol | 177 | 120 | nach 350 Std. steril* |
| Ethanol | 185 | keine | nach 350 Std. steril* |
| Ethanol | 198, 120 | keine | nach 350 Std. steril* |
| Aceton | 90 | 120 | nach 192 Std. kont. |
| Ethanolamin | 155 | 120 | nach 192 Std. kont. |
| ohne Quellen | 171 | 120 | nach 168 Std. kont. |
| Ethanol | keine | 184 | nach 144 Std. kont. |
| Ethanol | keine | 151, 120 | nach 144 Std. kont. |

| | | | |
|---|---|---|---|
| * Der Versuch wurde beendet, da das Probenmaterial zur Sterilitätsprüfung aufgebraucht war. | | | |

b) PU II (Tecothane® 1095 A,4-Liner):
- unbehandelter Kontrollkatheter: nach 55 Stunden kontaminiert
- silberimprägnierter Katheter (Quellung mit Aceton bzw. Ethanol, Ag-Lsg. gesättigt oder auch 6%ig): 168 Stunden lang steril*

c) PU III (Certofix® aus Tecoflex® EG 93 A B20):
- unbehandelter Kontrollkatheter: nach 15 Stunden kontaminiert
- silberimprägnierter Katheter (Quellung mit Aceton): 168 Stunden lang steril*

d) PU IV (Elastollan® 1195 A 10L + 25 % BaSO4):
- unbehandelter Kontrollkatheter: nach 5 Stunden kontaminiert
- silberimprägnierter Katheter (Quellung mit Aceton): nach 286 Stunden noch steril.*

* Der Versuch wurde beendet, da das Probenmaterial zur Sterilitätsprüfung aufgebraucht war.

### Silikon:

- unbehandelter Kontrollkatheter: nach 2 Stunden kontaminiert
- silberimprägnierter Katheter (Quellung mit Aceton): nach über 200 Stunden noch steril.*

In den Fällen, in denen während der Versuchsdauer eine Besiedelung stattfand, konnte im weiteren Verlauf der Versuch vielfach ein Rückgang der Besiedelungsdichte beobachtet werden, so daß die Katheter im Laufe der Zeit wieder "steriler" wurde. Auch dieser Effekt ist auf die besondere Verteilung der metallhaltigen Verbindung in dem Katheter zurückzuführen.

In begleitenden Kontrollversuchen zeigte sich auch, daß Katheter, die nur mit den zur Komplexierung verwendeten Aminen (z.B. Ethanolamin) behandelt wurden (d.h. ohne Silberverbindung) bereits leicht bakterizide Wirkung zeigen.
* Der Versuch wurde beendet, da das Probenmaterial zur Sterilitätsprüfung aufgebraucht war.

### Resultate der Gewebeverträglichkeitsuntersuchungen

Ergebnisse der Untersuchung der Gewebeverträglichkeit in Lymphozytenkulturen:

| | nach 48 Stunden | | nach 72 Stunden | |
|---|---|---|---|---|
| | OD 550 | Vitalität (%) | OD 550 | Vit. (%) |
| Certofix | 0,068 | 40,96 | 0,065 | 25,59 |
| Certofix* | 0,052 | 31,33 | 0,041 | 16,14 |
| Cavafix | 0,166 | 100 | 0,175 | 68,90 |
| Cavafix* | 0,145 | 87,35 | 0,136 | 53,54 |
| Tecothane | 0,119 | 71,69 | 0,134 | 52,76 |
| Tecothane* | 0,106 | 63,86 | 0,068 | 26,77 |
| Ellastollan | 0,075 | 45,18 | 0,095 | 37,40 |
| Ellastollan* | 0,055 | 33,13 | 0,019 | 7,48 |
| PHA-L. | 0,166 | 100 | 0,254 | 100 |

| | | | | |
|---|---|---|---|---|
| *silberimprägnierter Katheter | | | | |

Die Ergebnise der Untersuchung in Lymphozytenkulturen zeigen, daß eine Imprägnierung der Katheter mit Silber im Vergleich zu unbehandelten Katheter nur eine geringgradige Vitalitätsminderung PHA-stimulierter Lymphozyten bewirkt.
Die beste biologische Verträglichkeit zeigen die Katheter Cavafix und Tecothane.
Bemerkenswert aber ist, daß selbst unbehandelte Certofix- und Ellastollan-Katheter zu einer doch relativ hohen Absterberate von Lymphozyten geführt haben.

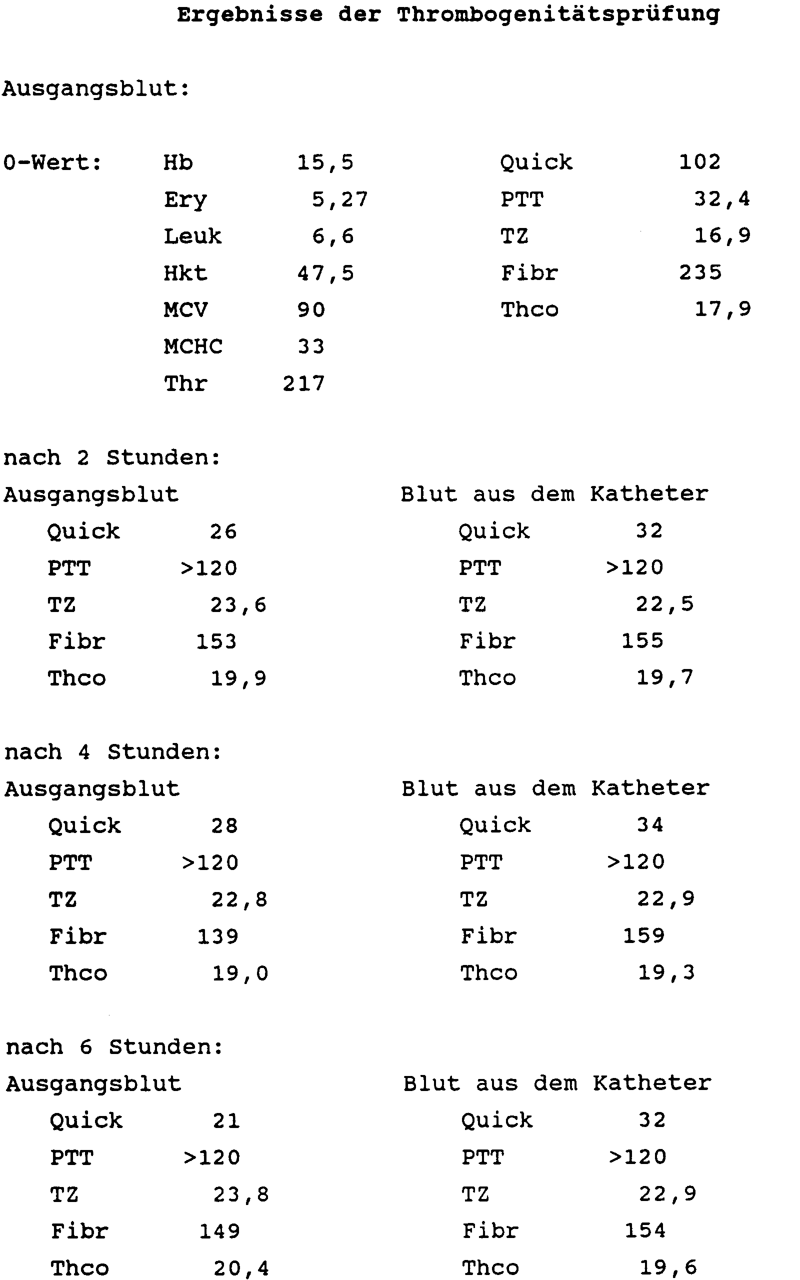

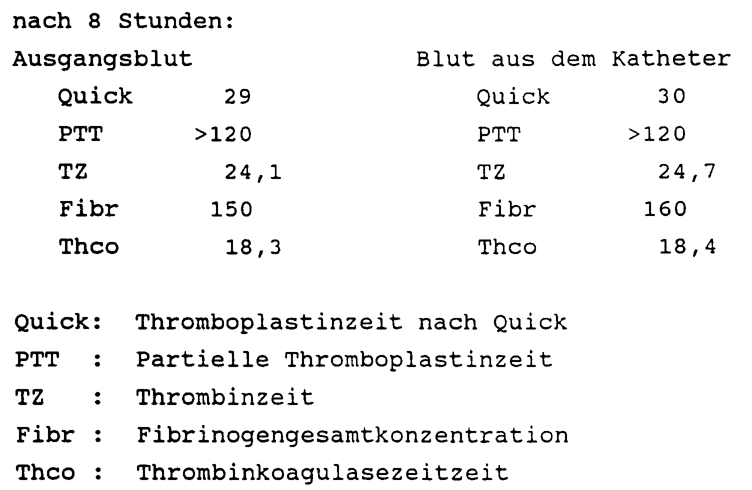

Die Ergebnisse der Thrombogenitätsprüfung zeigen, daß die Thrombinkoagulasezeit und die Fibrinogenkonzentration über den gesamten Untersuchungszeitraum konstant blieben, ebenso weitgehend die Thrombocytenzahl. In beiden Blutproben wies der Quick-Wert bei der Entnahme nach zwei Stunden Werte um 20-30 % auf, sank danach aber nicht mehr weiter ab. die Thrombinzeit war meist um 20 Sekunden und Partielle Thromboplastinzeit lag stets bei Werten über 120 Sekunden.
Da die Werte in beiden Blutproben immer nahezu identisch sind, läßt sich insgesamt feststellen, daß die Silberimprägnierung von Kathetern keinen Einfluß auf die Blutgerinnung hat und nicht thrombogen wirkt.

Dieses Verfahren bezüglich der Einbringung des Silberchlorides ist natürlich bezüglich verschiedener Parameter beeinflußbar und damit auch in wirtschaftlicher Hinsicht optimierbar. Grundsätzlich ist damit aber die Möglichkeit gegeben, die erfindungsgemäßen Produkte so herzustellen, daß sie für die Medizin einen wesentlichen Fortschritt bezüglich der Eindämmung des Risikos darstellen, bei ihrer Anwendung Patienten durch das Einschwemmen von Keimen zu schaden.

## Patentansprüche (Patentansprüche für folgende(n) Vertragsstaat(en): CH, DE, FR, GB, LI, NL)

1. Verfahren zur Herstellung eines nicht von Keimen und/oder Pilzen besiedelbaren Kunststoffteils, dadurch gekennzeichnet, daß der Kunststoff mit einem Quellungsmittel vorbehandelt wird und der erhaltene gequollene Kunststoff mit einer Lösung aus einer in Wasser unlöslichen Verbindung, die ein bakterizides und/oder fungizides Metall enthält, behandelt wird, ausgenommen Auranofin.

2. Verfahren nach Anspruch 1, wobei es sich bei der Verbindung, die ein bakterizides und/oder fungizides Metall enthält, um ein anorganisches Metallsalz handelt.

3. Verfahren nach Anspruch 1 oder 2, wobei das bakterizide und/oder fungizide Metall Silber, Kupfer, Gold, Zink oder Cer ist.

4. Verfahren nach einem der Ansprüche 1 bis 3, dadurch gekennzeichnet, daß das Lösungsmittel für die Verbindung, die ein bakterizides und/oder fungizides Metall enthält, das Metall komplexieren kann und wobei Auranofin verwendet werden kann.

5. Verfahren, nach einem der Ansprüche 1 bis 4, dadurch gekennzeichnet, daß der gequollene Kunststoff ohne vorheriges Trocknen mit einer Lösung einer Verbindung, die ein bakterizides und/oder fungizides Metall enthält, behandelt wird, wobei die Verbindung, die ein bakterizides und/oder fungizides Metall enthält, in dem Quellungsmittel für das Kunststoffteil schlechter löslich ist als in dem Lösungsmittel, in dem sie gelöst ist und wobei Auranofin verwendet werden kann.

6. Verfahren nach einen der Ansprüche 1 bis 5, dadurch gekennzeichnet, daß das Quellungsmittel Aceton oder Ethanol ist und wobei Auranofin verwendet werden kann.

7. Verfahren nach einem der Ansprüche 1 bis 6, dadurch gekennzeichnet, daß das Lösungsmittel ein organisches Amin oder Ammoniak enthält und wobei Auranofin verwendet werden kann.

8. Verfahren nach Anspruch 7, dadurch gekennzeichnet, daß das organische Amin ein Alkanolamin ist.

9. Verfahren nach Anspruch 8, dadurch gekennzeichnet, daß das Alkanolamin Ethanolamin ist.

10. Verfahren nach einem der Ansprüche 1 bis 9, dadurch gekennzeichnet, daß die Verbindung, die ein bakterizides und/oder fungizides Metall enthält Silberchlorid ist und das Lösungsmittel für diese Verbindung Ethanolamin ist.

11. Verfahren nach einem der Ansprüche 1-4 und 7-10, dadurch gekennzeichnet, daß das Kunststoffteil Polyethylen, Polypropylen, Polyurethan, Silikon, allein oder in Verbindung miteinander enthält.

12. Verfahren nach einem der Ansprüche 1-4 und 7-10, dadurch gekennzeichnet, daß das Kunststoffteil ein Vorprodukt für medizinische Geräte ist.

13. Verfahren nach einem der Ansprüche 5 und 6, dadurch gekennzeichnet, daß das Kunststoffteil Polyethylen, Polypropylen, Polyurethan, Silikon, allein oder in Verbindung miteinander enthält.

14. Verfahren nach einem der Ansprüche 5 und 6, dadurch gekennzeichnet, daß das Kunststoffteil ein Vorprodukt für medizinische Geräte ist.

15. Nicht von Keimen besiedelbares Kunststoffteil, erhältlich nach dem Verfahren nach einem der Ansprüche 1 bis 4 und 7 bis 12.

## Patentansprüche (Patentansprüche für folgende(n) Vertragsstaat(en): AT)

1. Verfahren zur Herstellung eines nicht von Keimen und/oder Pilzen besiedelbaren Kunststoffteils, dadurch gekennzeichnet, daß der Kunststoff mit einem Quellungsmittel vorbehandelt wird und der erhaltene gequollene Kunststoff mit einer Lösung aus einer in Wasser unlöslichen Verbindung, die ein bakterizides und/oder fungizides Metall enthält, behandelt wird.

2. Verfahren nach Anspruch 1, wobei es sich bei der Verbindung, die ein bakterizides und/oder fungizides Metall enthält, um ein anorganisches Metallsalz handelt.

3. Verfahren nach Anspruch 1 oder 2, wobei das bakterizide und/oder fungizide Metall Silber, Kupfer, Gold, Zink oder Cer ist.

4. Verfahren nach einem der Ansprüche 1 bis 3, dadurch gekennzeichnet, daß das Lösungsmittel für die Verbindung, die ein bakterizides und/oder fungizides Metall enthält, das Metall komplexieren kann.

5. Verfahren, insbesondere nach einem der Ansprüche 1 bis 4, dadurch gekennzeichnet, daß der gequollene Kunststoff ohne vorheriges Trocknen mit einer Lösung einer Verbindung, die ein bakterizides und/oder fungizides Metall enthält, behandelt wird, wobei die Verbindung, die ein bakterizides und/oder fungizides Metall enthält, in dem Quellungsmittel für das Kunststoffteil schlechter löslich ist als in dem Lösungsmittel, in dem sie gelöst ist.

6. Verfahren nach einem der Ansprüche 1 bis 5, dadurch gekennzeichnet, daß das Quellungsmittel Aceton oder Ethanol ist.

7. Verfahren nach einem der Ansprüche 1 bis 6, dadurch gekennzeichnet, daß das Lösungsmittel ein organisches Amin oder Ammoniak enthält.

8. Verfahren nach Anspruch 7, dadurch gekennzeichnet, daß das organische Amin ein Alkanolamin ist.

9. Verfahren nach Anspruch 8, dadurch gekennzeichnet, daß das Alkanolamin Ethanolamin ist.

10. Verfahren nach einem der Ansprüche 1 bis 9, dadurch gekennzeichnet, daß die Verbindung, die ein bakterizides und/oder fungizides Metall enthält Silberchlorid ist und das Lösungsmittel für diese Verbindung Ethanolamin ist.

11. Verfahren nach einem der Ansprüche 1 bis 10, dadurch gekennzeichnet, daß das Kunststoffteil Polyethylen, Polypropylen, Polyurethan, Silikon, allein oder in Verbindung miteinander enthält.

12. Verfahren nach einem der Ansprüche 1 bis 11, dadurch gekennzeichnet, daß das Kunststoffteil ein Vorprodukt für medizinische Geräte ist.

13. Nicht von Keimen besiedelbares Kunststoffteil, erhältlich nach dem Verfahren nach einem der Ansprüche 1 bis 12.

## Claims (Claims for the following Contracting State(s): CH, DE, FR, GB, LI, NL)

1. Process for producing a plastic part that cannot be colonised by germs and/or fungi, characterized in that the plastic is pre-treated with a swelling agent and that the obtained swollen plastic is treated with a solution of a water-insoluble compound containing a bactericidal and/or fungicidal metal with the exception of auranofin.

2. Process of claim 1, wherein the compound containing a bactericidal and/or fungicidal metal is an inorganic metal salt.

3. Process of claim 1 or 2, wherein the bactericidal and/or fungicidal metal is silver, copper, gold, zinc or cerium.

4. Process of any one of claims 1 to 3, characterized in that the solvent for the compound containing a bactericidal and/or fungicidal metal can complex the metal and that auranofin may be used.

5. Process of any one of claims 1 to 4, characterized in that the swollen plastic is treated with a solution of a compound containing a bactericidal and/or fungicidal metal without being dried first, and wherein the compound containing a bactericidal and/or fungicidal metal is not as readily soluble in the swelling agent for the plastic as in the solvent in which it is dissolved and wherein auranofin may be used.

6. Process of any one of claims 1 to 5, characterized in that the swelling agent is acetone or ethanol and that auranofin may be used.

7. Process of any one of claims 1 to 6, characterized in that the solvent contains an organic amine or ammonia and that auranofin may be used.

8. Process of claim 7, characterized in that the organic amine is an alkanol amine.

9. Process of claim 8, characterized in that the alkanol amine is ethanol amine.

10. Process of any one of claims 1 to 9, characterized in that the compound containing a bactericidal and/or fungicidal metal is silver chloride and the solvent for this compound is ethanol amine.

11. Process of any one of claims 1 to 4 and 7 to 10, characterized in that the plastic part contains polyethylene, polypropylene, polyurethane, silicone, alone or in combination.

12. Process of any one of claims 1 to 4 and 7 to 10, characterized in that the plastic part is a preproduct for medical devices.

13. Process of any one of claims 5 and 6, characterized in that the plastic part contains polyethlyene, polypropylene, polyurethane, silicone, alone or in combination.

14. Process of any one of claims 5 and 6, characterized in that the plastic part is a preproduct for medical devices.

15. Plastic part that cannot be colonised by germs and is obtained in a process of any one of claims 1 to 4 and 7 to 12.

## Claims (Claims for the following Contracting State(s): AT)

1. Process for producing a plastic part that cannot be colonised by germs and/or fungi, characterized in that the plastic is pre-treated with a swelling agent and that the obtained swollen plastic is treated with a solution of a water-insoluble compound containing a bactericidal and/or fungicidal metal.

2. Process of claim 1, wherein the compound containing a bactericidal and/or fungicidal metal is an inorganic metal salt.

3. Process of claim 1 or 2, wherein the bactericidal and/or fungicidal metal is silver, copper, gold, zinc or cerium.

4. Process of any one of claims 1 to 3, characterized in that the solvent for the compound containing a bactericidal and/or fungicidal metal can complex the metal.

5. Process, particularly of any one of claims 1 to 4, characterized in that the swollen plastic is treated with a solution of a compound containing a bactericidal and/or fungicidal metal without being dried first, and wherein the compound containing a bactericidal and/or fungicidal metal is not as readily soluble in the swelling agent for the plastic as in the solvent in which it is dissolved.

6. Process of any one of claims 1 to 5, characterized in that the swelling agent is acetone or ethanol.

7. Process of any one of claims 1 to 6, characterized in that the solvent contains an organic amine or ammonia.

8. Process of claim 7, characterized in that the organic amine is an alkanol amine.

9. Process of claim 8, characterized in that the alkanol amine is ethanol amine.

10. Process of any one of claims 1 to 9, characterized in that the compound containing a bactericidal and/or fungicidal metal is silver chloride and the solvent for this compound is ethanol amine.

11. Process of any one of claims 1 to 10, characterized in that the plastic part contains polyethylene, polypropylene, polyurethane, silicone, alone or in combination.

12. Process of any one of claims 1 to 11, characterized in that the plastic part is a preproduct for medical devices.

13. Plastic part that cannot be colonised by germs and is obtained in a process of any one of claims 1 to 12.

## Revendications (Revendications pour l'(les) Etat(s) contractant(s) suivant(s): CH, DE, FR, GB, LI, NL)

1. Procédé de préparation d'une pièce en matière synthétique qui ne peut pas être colonisée par des germes et/ou des champignons, caractérisé en ce que la matière synthétique est prétraitée avec un agent de gonflement et la matière synthétique gonflée obtenue est traitée avec une solution d'un composé insoluble dans l'eau qui contient un métal bactéricide et/ou fongicide, à l'exception de l'auranofine.

2. Procédé selon la revendication 1 dans lequel le composé qui contient un métal bactéricide et/ou fongicide est un sel métallique inorganique.

3. Procédé selon la revendication 1 ou 2, dans lequel le métal bactéricide et/ou fongicide est l'argent, le cuivre, l'or, le zinc ou le cérium.

4. Procédé selon l'une des revendications 1 à 3, caractérisé en ce que le solvant pour le composé qui contient un métal bactéricide et/ou fongicide peut complexer le métal et où l'auranofine peut être utilisée.

5. Procédé selon l'une des revendications 1 à 4, caractérisé en ce que la matière synthétique gonflée est traitée sans séchage préalable avec une solution d'un composé qui contient un métal bactéricide et/ou fongicide, où le composé qui contient un métal bactéricide et/ou fongicide est moins soluble dans l'agent de gonflement pour la pièce en matière synthétique que dans le solvant dans lequel il est dissous, et où l'auranofine peut être utilisée.

6. Procédé selon l'une des revendications 1 à 5, caractérisé en ce que l'agent de gonflement est l'acétone ou l'éthanol et où l'auranofine peut être utilisée.

7. Procédé selon l'une des revendications 1 à 6, caractérisé en ce que le solvant contient une amine organique ou de l'ammoniac et où l'auranofine peut être utilisée.

8. Procédé selon la revendication 7, caractérisé en ce que l'amine organique est une alcanolamine.

9. Procédé selon la revendication 8, caractérisé en ce que l'alcanolamine est l'éthanolamine.

10. Procédé selon l'une des revendications 1 à 9, caractérisé en ce que le composé qui contient un métal bactéricide et/ou fongicide est le chlorure d'argent et le solvant pour ce composé est l'éthanolamine.

11. Procédé selon l'une des revendications 1 à 4 et 7 à 10, caractérisé en ce que la pièce en matière synthétique contient du polyéthylène, du polypropylène, du polyuréthane, une silicone, seuls ou en combinaison les uns avec les autres.

12. Procédé selon l'une des revendications 1 à 4 et 7 à 10, caractérisé en ce que la pièce en matière synthétique est une ébauche pour appareils médicaux.

13. Procédé selon l'une des revendications 5 et 6, caractérisé en ce que la pièce en matière synthétique contient du polyéthylène, du polypropylène, du polyuréthane, une silicone, seuls ou en combinaison les uns avec les autres.

14. Procédé selon l'une des revendications 5 et 6, caractérisé en ce que la pièce en matière synthétique est une ébauche pour appareils médicaux.

15. Pièce en matière synthétique qui ne peut pas être colonisée par des germes, qui peut être obtenue par le procédé selon l'une des revendications 1 à 4 et 7 à 12.

## Revendications (Revendications pour l'(les) Etat(s) contractant(s) suivant(s): AT)

1. Procédé de préparation d'une pièce en matière synthétique qui ne peut pas être colonisée par des germes et/ou des champignons, caractérisé en ce que la matière synthétique est prétraitée avec un agent de gonflement et la matière synthétique gonflée obtenue est traitée avec une solution d'un composé insoluble dans l'eau qui contient un métal bactéricide et/ou fongicide.

2. Procédé selon la revendication 1 où le composé qui contient un métal bactéricide et/ou fongicide est un sel métallique inorganique.

3. Procédé selon la revendication 1 ou 2 où le métal bactéricide et/ou fongicide est l'argent, le cuivre, l'or, le zinc ou le cérium.

4. Procédé selon l'une des revendications 1 à 3, caractérisé en ce que le solvant pour le composé qui contient un métal bactéricide et/ou fongicide peut complexer le métal.

5. Procédé, en particulier selon l'une des revendications 1 à 4, caractérisé en ce que la matière synthétique gonflée est traitée sans séchage préalable avec une solution d'un composé qui contient un métal bactéricide et/ou fongicide, où le composé qui contient un métal bactéricide et/ou fongicide est moins soluble dans l'agent de gonflement pour la pièce en matière synthétique que dans le solvant dans lequel il est dissous.

6. Procédé selon l'une des revendications 1 à 5, caractérisé en ce que l'agent de gonflement est l'acétone ou l'éthanol.

7. Procédé selon l'une des revendications 1 à 6, caractérisé en ce que le solvant contient une amine organique ou de l'ammoniac.

8. Procédé selon la revendication 7, caractérisé en ce que l'amine organique est une alcanolamine.

9. Procédé selon la revendication 8, caractérisé en ce que l'alcanolamine est l'éthanolamine.

10. Procédé selon l'une des revendications 1 à 9, caractérisé en ce que le composé qui contient un métal bactéricide et/ou fongicide est le chlorure d'argent et le solvant pour ce composé est l'éthanolamine.

11. Procédé selon l'une des revendications 1 à 10, caractérisé en ce que la pièce en matière synthétique contient du polyéthylène, du polypropylène, du polyuréthane, une silicone, seuls ou en combinaison les uns avec les autres.

12. Procédé selon l'une des revendications 1 à 11, caractérisé en ce que la pièce en matière synthétique est une ébauche pour appareils médicaux.

13. Pièce en matière synthétique qui ne peut pas être colonisée par des germes, qui peut être obtenue par le procédé selon l'une des revendications 1 à 12.
